(19) Europäisches Patentamt
European Patent Office
Office européen des brevets

(11) Numéro de publication: **0 353 145**
**A1**

(12) # DEMANDE DE BREVET EUROPEEN

(21) Numéro de dépôt: **89402105.4**

(22) Date de dépôt: **25.07.89**

(51) Int. Cl.$^5$: **C 12 P 19/04**
C 12 N 1/20
//C12R1:38

(30) Priorité: **25.07.88 FR 8809999**

(43) Date de publication de la demande:
**31.01.90 Bulletin 90/05**

(84) Etats contractants désignés:
**AT BE CH DE ES FR GB GR IT LI LU NL SE**

(71) Demandeur: **CENTRE NATIONAL DE LA RECHERCHE SCIENTIFIQUE (CNRS)**
**15, Quai Anatole France**
**F-75700 Paris Cedex 07 (FR)**

(72) Inventeur: **Balandreau, Jacques**
**24, rue V. Basch**
**F-54500 Vandoeuvre Les Nancy (FR)**

**Gueniot, Bernard**
**42, rue Mai Exelmans**
**F-54000 Nancy (FR)**

**Hebbar, Prakash**
**Hebbar Brothers**
**Palghai 678007, Kerala (IN)**

(74) Mandataire: **Warcoin, Jacques et al**
**Cabinet Régimbeau 26, avenue Kléber**
**F-75116 Paris (FR)**

Le microorganisme a été déposé auprès la Collection Nationale de Cultures de Microorganismes (CNCM) sous le numéro I-886.

(54) **Polysaccharides, procédé pour leur préparation par culture de pseudomonas paucimobilis et application des souches en agriculture.**

(57) La présente invention concerne un procédé de préparation d'exopolysaccharides, caractérisé en ce qu'on cultive sur un milieu de culture une souche de Pseudomonas paucimobilis et en ce qu'on récupère les exopolysaccharides du milieu de culture et l'utilisation de cette espèce bactérienne et/ou de son produit en agronomie et dans l'industrie alimentaire.

EP 0 353 145 A1

## Description

### POLYSACCHARIDES, PROCEDE POUR LEUR PREPARATION PAR CULTURE DE PSEUDOMONAS PAUCIMOBILIS ET APPLICATION DES SOUCHES EN AGRICULTURE

La présente invention concerne la préparation de nouveaux polysaccharides par les bactéries Pseudomonas paucimobilis, ainsi que l'utilisation de ces bactéries dans l'agriculture.

La présente invention repose sur la mise en évidence des propriétés particulièrement intéressantes des bactéries P. paucimobilis, notamment en tant qu'organismes producteurs de polysaccarides, mais également comme agents présentant une activité nitrogénasique importante.

P. paucimobilis, en particulier la souche RMDP17, produit un polysaccaride capsulaire (lié à la cellule) et non capsulaire (relargué dans le milieu) en quantités importantes (1 à 2 g/l en matière sèche).

Ce polysaccaride peut éventuellement être utilisé pour remplacer le xanthane comme agent épaississant dans ses nombreuses applications, en agro-alimentaire, pharmacie et cosmétique notamment, ceci tenant compte du comportement rhéologique très particulier du polysaccaride selon l'invention, à savoir notamment que sa viscosité est assez peu sensible à la force ionique du milieu et à la température.

La présente invention concerne, tout d'abord, un exopolysaccharide produit par P. paucimobilis dont la chaîne saccaridique est la suivante :

$$\left[\ 4\ \mathrm{Glc}\ 1 \longrightarrow 3\ \underset{\substack{\uparrow 6\\ \mathrm{Glc\ B\acute{e}ta1}}}{\mathrm{Glc}}\ \mathrm{B\acute{e}ta1} \longrightarrow 6\ \mathrm{Glc\ Alpha1} \longrightarrow 4\ \mathrm{Rha}\ 1\ \right]_n$$

n représente le nombre d'unités ; bien qu'il soit difficile d'évaluer le poids moléculaire, généralement, n est compris entre 500 et 10 000, et de préférence entre 1 000 et 4 000.

Il s'agit d'un polysaccaride neutre. Il et légèrement acétylé mais ne comporte pas de radical pyruvyl ou succinyl et contient, en général, moins de 10 % d'acide uronique en poids de matière sèche.

D'autres caractéristiques du polysaccaride selon l'invention seront étudiées plus en détail dans les exemples.

La présente invention concerne également un procédé de préparation de ce polysaccaride contenant moins de 10 % d'acide uronique, caractérisé en ce qu'on cultive sur un milieu de culture une souche identifiée comme P. paucimobilis et en ce qu'on récupère les exopolysaccarides du milieu de culture.

P. paucimobilis, en particulier la souche RMDP17, n'a pas d'exigences trophiques particulières. Elle pousse très bien sur la plupart des milieux de culture tels que le bouillon nutritif, la gélose nutritive, le "Luria broth" et les milieux pour bactéries fixatrices d'azote comme le milieu de Döbereiner et Day, le milieu de Watanabe ou le milieu RCV dont la préparation est décrite dans les exemples.

Les études d'optimisation des milieux de culture ont montré que les sources de carbone les plus intéressantes pour la préparation d'exopolysaccarides étaient les aldohexoses, glucose notamment, et que la concentration en glucose n'était pas un élément limitatif. Par contre, la production d'exopolysaccharides est sensiblement proportionnelle à la disponibilité en azote combiné.

Les autres conditions de culture ne constituent pas des éléments caractéristiques de la présente invention, il s'agit d'une bactérie aérobie qui peut croître à des températures pouvant varier de 18 à 37°C.

L'une des caractéristiques tout à fait intéressante du procédé selon l'invention est l'utilisation de prédateurs tels que les amibes, les bactéries prédatrices, les mixobactéries, les champignons, les phages, les protozoaires, les nématodes et les leucocytes, afin d'augmenter la production d'exopolysaccharides.

Il semble que l'utilisation de ces prédateurs permette une sélection de mutants hyperproducteurs de polysaccharides. Cette opération peut être conduite séparément pour isoler un mutant hyperproducteur ou bien être utilisée comme une pression de sélection dans le milieu de culture.

Le polysaccharide en cause peut être utilisé en remplacement du xanthane comme agent gélifiant et épaississant mais peut être également utilisé comme agent de conditionnement des sols, comme cela sera décrit ci-après dans les exemples.

En outre, P. paucimobilis, en particulier RMDP17, présente des propriétés particulièrement intéressantes dans le domaine agricole de la culture du mil. En effet, les bactéries selon l'invention présentent un fort pouvoir colonisateur des racines du mil : après bactérisation des graines, elle est capable de croître et de se multiplier le long des racines où elle peut représenter après 7 jours 10 % de la microflore totale (expérience en pots, au laboratoire, sur sol venu de la station de Bambey au Sénégal).

En inoculant les semences du mil à l'aide de ces bactéries, on peut envisager d'augmenter la fixation d'azote et donc d'améliorer la croissance. De même, ce fort pouvoir colonisateur et la production de sidérophore doivent permettre de lutter contre les pathogènes du mil.

L'inoculation peut s'effectuer au niveau de la semence, par exemple par enrobage, mais on peut imaginer d'inoculer les bactéries dans le sol avec ou sans support, selon des procédés connus.

Concernant la souche RMDP17, il faut citer également la propriété de donner, sur milieu dépourvu d'azote combiné, à la fois des colonies jaunes brillantes extrêmement surélevées présentant une forte activité nitrogénasique et des colonies blanches brillantes appliquées sur le substrat, et dépourvues d'activité nitrogénasique.

D'autres caractéristiques et avantages de l'invention apparaîtront à la lecture des exemples ci-après qui se réfèrent aux dessins ci-annexés sur lesquels :

. la figure 1 représente l'évolution de la viscosité relative en fonction du gradient de cisaillement, comportement comparés du produit de la souche RMDP17 et du xanthane (Rhodopol RAP 23-1976), mesure en viscosimètre "low shear" ;

. la figure 2 est un graphique montrant l'influence de la présence d'un prédateur (une amibe) sur la production d'exopolysaccharide en fonction de la concentration en tampon ;

. la figure 3 est un graphique montrant, en fonction du temps, l'influence des exopolysaccarides de la souche RMDP17 par rapport à un succinoglycane produit par la souche GM1848 sur le pourcentage d'agrégats stables.

## EXEMPLE 1

La souche de P. paucimobilisdénommée RMDP17 a été isolée de la rhizosphère du mil.

Il s'agit d'une bactérie Gram négative, en bâtonnets mobiles. oxydase négative, catalase positive ; le profil API 20 NE est 0463 (3/7) (4/6) 4 ; le profil 20 EC est 0 210 005 ; les colonies, en condition de fixation d'azote, sont jaunes.

Un antisérum préparé sur lapin contre cette souche donne une réaction croisée forte avec la souche 5 Aj. Cette dernière, par hybridation ADN/ARNr, s'est avérée très proche de Flavobacterium capsulatum ATCC 14 666 (Bally et coll.).

Cet antisérum ne présente pas de réaction croisée avec P. fluorescens souche W et P. Cepacia.

Cette souche, appelée RMDP17, est très semblable à P. paucimobilis, taxon défini par Holmes et coll. Il faut remarquer qu'il s'agit d'un taxon mal nommé, éloigné de la plupart des espèces de Pseudomonas qui appartiennent aux groupes II et III de De ley ; P. paucimobilis se rattache en fait au group IV défini par De Ley. En tant que telle, cette bactérie apparaît apparentée à Flavobacterium capsulatum, Azospirillum, Rhizobium, Agrobacterium.

Cette souche a été cultivée sur un milieu dont la préparation est décrite ci-dessous :

Trois solutions doivent être préparées :

1. Solution "éléments" (mg/l d'eau distillée)

| | |
|---|---|
| Zn $SO_4$ $7H_2O$ | 430 |
| Mn $SO_4$ $H_2O$ | 1300 |
| Na Mo $O_4$ $2H_2O$ | 750 |
| $H_3$ $BO_3$ | 2800 |
| Cu $SO_4$ $7H_2O$ | 26 |
| Co $SO_4$ $7H_2O$ | 70 |
| Eau distillée | 1000 ml |

2. Solution "Supersalts" (mg/l d'eau distillée)

| | |
|---|---|
| EDTA | 400 |
| $MgSO_4$ $7H_2O$ | 2000 |
| $CaCl_2$ $2H_2O$ | 2000 |
| $FeSO_4$ $7H_2O$ | 440 |
| Solution "éléments" | 20 ml |
| Eau distillée | 1000 ml |

3. Tampon phosphate

| | |
|---|---|
| $KH_2PO_4$ | 40 g |
| $K_2HPO_4$ | 60 g |
| Eau distillée | 1000 ml |

Milieu final :

| | |
|---|---|
| Solution "Supersalts" | 50 ml |
| Tampon phosphate | 15 ml |
| Substrat carboné | 1 à 10 g/l |
| Eau distillée, qsp | 1000 ml |
| Extrait de levure | 0,1 g/l |

Note: Le substrat carboné peut être un sucre ou du malate. Ce milieu est une modification de celui utilisé par Weaver et et coll. pour Rhodopseudomonas.

Après culture, les exopolysaccharides sont séparés comme décrit ci-après :

Les exopolysaccharides produits sont précipitables à l'éthanol, apporté à raison de 1,5 volumes par volume de moût ; ils sont alors récupérables par centrifugation (5000 g pendant 15 à 20 minutes) ou par filtration sur une série de filtres Millipore de taille décroissante (5 ; 2 puis 0,45 microns). Ils peuvent alors être déshydratés sur le filtre par des solutions d'éthanol de plus en plus concentrées (60 ; 75 puis 95°) et séchés à l'étuve à 35°C pendant 48 heures.

## EXEMPLE2

### Etude des propriétés du polysaccharide

Analyse chimique :

. Composition en sucres neutres et acides

Après hydrolyse partielle (1 ml d'acide sulfurique molaire pour 100 mg d'exopolysaccharides) la composition de l'hydrolysat a été etudiée par GLC des méthylglycosides trifluoroacétylés. Deux monosaccharides ont été identifiés : le glucose et le rhamnose, dans les rapports molaires 3/1 ou 4/1. Sur le même hydrolysat, les sucres et substituants ont été identifiés et dosés par chromotographie sur colonne Aminex ion exclusion HPX-87 H(300x7,8 mm Biorad) : sur le chromatogramme apparaissent seulement deux constituants (glucose et rhamnose) et des traces d'acide acétique. La détection s'est faite grâce à un réfractiomètre differentiel. La confirmation des substituants acides s'est faite sur colonne de micro-Bondapak C18 modifiée par acétylation.

. Analyse de l'enchaînement des monosaccharides par perméthylation

Le polysaccharide a été perméthylé selon la méthode de Paz-Parente et coll. (1985) et les éthers méthyliques libérés par méthanolyse, analysés par GLC selon la méthode de Fournet et coll. (1981). La structure proposée est la suivante :

$$\left[ 4\ \mathrm{Glc}\ 1 \longrightarrow 3\ \mathrm{Glc\ Beta1} \longrightarrow 6\ \mathrm{Glc\ Alpha1} \longrightarrow 4\ \mathrm{Rha}\ 1 \right]_n$$

(sur le 3 Glc Beta1 : ↑ 6 — Glc Béta1)

. Substituants acides

Leur quantification a été faite par RMN du proton. Seuls des substituants acétyle ont pu être mis en évidence. Ils sont très peu nombreux. Il n'y a pas de substituants succinyl ou pyruvyl.

Etude des propriétés physiques

. Pouvoir rotatoire et température de transition conformationnelle

Le produit est soluble à froid dans l'eau. Son pouvoir rotatoire est

$(\text{alpha})_{300}^{25} = -84.$

Il n'y a pas de transition conformationnelle entre 10 et 90° C. Le pouvoir rotatoire a été établi sur des solutions d'exopolysaccharides à 1 g/l dans NaCl 0,1 M, à 300 nm avec un spectropolarimètre (FICA Spectrol 1).

. Propriétés rhéologiques

Ce polysaccharide est doué d'une grande viscosité supérieure à celle du xanthane (figure 1). Cette propriété est relativement plus stable que celle du xanthane lorsque la force ionique augmente ou lorsque le pH est acide ; cette viscosité est peu affectée par un chauffage de 10 minutes à 90° C.

Tableau 1

Comparaison des viscosités relatives du produit de la souche RMDP17 et du xanthane

(Solutions à 1 g/l, viscosité mesurée à l'aide d'un viscosimètre "low-shear" pour un gradient de cisaillement gamma = $0{,}08s^{-1}$)

| Milieu | Eau | NaCl (0,1 M) | NaCl (1,7 M) | CaCl2 (0,3 M) | HCl (0,5 N) |
|---|---|---|---|---|---|
| RMDP17 | 1613 | 2477 | 2440 | 2558 | 1186 |
| Xanthane (Rhodopol RAP 23-1976) | 626 | 194 | 223 | 164 | 32 |

Tableau 2

Evolution de la viscosité relative à gamma = $0{,}94\ s^{-1}$ du produit de la souche RMDP17 en fonction du temps de chauffage à 90° C

| Temps de chauffage | 0 | 10 min. | 24 h | 48 h |
|---|---|---|---|---|
| Viscosité relative | 472 | 406 | 58 | 9 |

Les études ont été réalisées à l'aide d'un rhéomètre "low shear" 30. Outre les propriétés déjà mentionnées, il faut noter que le polysaccharide a sa viscosité qui augmente après chauffage à 90° C dans NaCl ; si elle diminue peu en milieu acide, par contre elle disparaît totalement en milieu basique.

EXEMPLE 3

Les études suivantes ont permis de mettre en évidence certains éléments intéressants de la culture de la souche en cause.

Source de carbone

Le tableau ci-dessous résume les résultats obtenus lors d'une étude de l'influence de diverses sources de carbone sur la croissance et la production de polysaccharides mise en évidence par la fluorescence en présence de "Calcofluor White".

Tableau 3

| Source de carbone | | Croissance | Fluorescence |
|---|---|---|---|
| Aldohexoses : | Glucose | ++ | ++ |
| | Sucrose | ++ | ++ |
| | Fructose | ++ | ++ |
| Aldopentoses : | Arabinose | + | + |
| | Xylose | + | + |
| | Maltose | + | - |
| Polyols : | Mannitol | (+) | + |
| | Sorbitol | (+) | + |
| | Glycérol | (+) | + |
| Acides organiques: | Citrate | (+) | + |
| | Malate | + | + |
| | Succinate | + | - |

Tableau 3 (suite)

| | | | |
|---|---|---|---|
| Acides aminés : | L-Arginine | 0 | 0 |
| | L-Glycine | (+) | 0 |
| | Méthionine | (+) | 0 |
| | Glutamine | (+) | 0 |
| | L-Leucine | + | 0 |
| | Aspargine | + | 0 |
| | L-Alanine | + | 0 |
| | Tyrosine | + | 0 |

La production est maximale sur les aldohexoses. Les études de concentration montre que la concentration en glucose n'est pas un facteur limitant de la production de polysaccharide et que la production est sensiblement la même pour 0,5 à 3 g/100 ml de glucose.

Une étude a été faite de l'influence de la concentration en tampon phosphate du milieu RCV ; elle montre que 40 ml/l est une concentration encore insuffisante.

La facteur le plus important pour la production de polysaccharide est sans doute la nutrition azotée ; contrairement à ce qui est connu chez de nombreuses bactéries la production d'exopolysaccharides est ici proportionnelle à la disponibilité en azote combiné.

Une étude de quelques mutants spontanés de résistance à la rifampicine montre qu'il existe probablement un contrôle transcriptionnel de la quantité d'exopolysaccharide synthétisée. Il est donc intéressant d'utiliser certains mutants résistant à la rifampicine pour préparer les exopolysaccharides.

## EXEMPLE 4

Des cultures en présence de prédateurs, en particulier en présence d'une amibe du sol, ont permis de mettre en évidence les avantages de ce type de culture.

La présence de prédateurs modifie souvent le niveau des populations bactériennes et peut induire des modifications, en particulier au niveau des enveloppes, mais il semble que cette propriété n'a jamais été utilisée pour sélectionner des mutants hyperproducteurs de matériel capsulaire ; le graphique de la figure 2 montre que la souche RMDP17 augmente fortement sa production d'exopolysaccharides lorsqu'on la met en présence d'une amibe du sol (Acanthamoeba sp.).

L'utilisation de prédateurs comme moyen de sélection de mutants hyperproducteurs de polysaccharides constitue un élément important de la présente invention.

Le terme "prédateur" s'entend de la façon la plus large et inclut toutes les bactéries prédatrices, les mixobactéries, les champignons, les phages, les protozoaires, les nématodes et les leucocytes.

## EXEMPLE 5

La souche RMDP17 a été isolée de la rhizosphère d'un mil poussant sur un sol ferralitique de l'Andhra Pradesh, aux Indes. Le pouvoir colonisateur sur cette plante a été évalué à l'aide d'une expérience très simple : du mil, c.v. Souna, a été inoculé au niveau des semences par trempage dans une culture dense de la souche et mis à pousser sur du sol Dior de la station agronomique de Bambey au Sénégal, pendant trois semaines au laboratoire. Au bout de ce temps, la bactérie avait bien colonisé le système racinaire du mil, où elle représentait environ 12 % de la microflore bactérienne totale. Cette espèce bactérienne a donc un certain potentiel comme inoculum du mil à des fins variées, pouvant aller de l'augmentation de sa nutrition minérale, de sa croissance, à la lutte contre ses pathogènes majeurs, mineurs, ou les bactéries délétères. On pourrait envisager aussi d'utiliser cette bactérie après manipulation génétique pour introduire dans la rhizosphère du mil des gènes utiles à l'homme. Enfin, la production de grandes quantités de polysaccharides hydrophiles par Pseudomonas paucimobilis pourrait éventuellement être mise à profit pour augmenter le stock d'eau disponible au niveau de la rhizosphère et permettre une meilleure résistance du mil ou d'autres plantes au stress hydrique.

Une étude a été réalisée de l'effet d'une addition au sol du polysaccharide produit par Pseudomonas paucimobilis RMDP17. La figure 3 montre la comparaison de l'effet du produit de la souche RMDP17 avec celui d'un succinoglycane de type classique produit par une souche d'Agrobacterium radiobacter, la souche GM 1848. Le pourcentage d'agrégats stables au benzène a été mesuré par la méthode classique de Hénin. Les échantillons de sol (sol argilo-limoneux des environs de Nancy) ont été arrosés à la capacité au champ au début de l'expérience, après ajout des polysaccharides, puis incubés à l'air. Ils se sont desséchés progressivement au cours de l'incubation et au 20ème jour l'humidité a été réajustée à la capacité au champ ; les échantillons traités avec le produit de souche RMDP17 montre alors un comportement singulier : sa structuration augmente pour redevenir égale à celle observée un début de l'expérience, si ce n'est supérieure (figure 3).

Cette propriété est très inhabituelle et pourrait être mise à profit pour utiliser les polysaccharides de P. paucimobilis comme conditionneurs de sols ou comme additif dans toutes les situations où le sol est utilisé comme ingrédient dans la réalisation d'un produit dont les propriétés mécaniques sont un caractère essentiel. Les applications pourraient s'envisager dans différents domaines, de l'agriculture (amélioration des sols battants) au génie civil (consolidation de talus).

La souche Pseudomonas paucimobilis RMDP17 a été déposée le 7 juillet 1989 à la Collection Nationale de Cultures de Microorganismes (CNCM) de l'Institut Pasteur, 28 rue du Docteur-Roux - 75724 Paris Cédex 15, sous le n° I-886, conformément aux dispositions du Traité de Budapest.

## BIBLIOGRAPHIE

Bally R., Thomas-Bauzon D., Heulin T., Balandreau J., Richard C., De Ley J., 1983, Determination of the most frequent N2-fixing bacteria in a rice rhizosphere. Can. J. Microbiol., 28,881-887.

De Ley J., 1981. Evolution des cistrons codant pour l'ARNr bactérien. Symbioses, 13, 109-121.

Döbereiner J. et Day J.M., 1976, Associative symbioses in tropical grasses: characterization of microorganisms and dinitrogen fixing sites. In: Nitrogen fixation, edité par W.E. Newton et C.J. Nyman. Washington State University Press, Pullman, W.A. pp. 518-538.
Holmes B., Owen R.J., Evans A., Malnick H., Willcox W.R., 1977. Pseudomonas paucimobilis, a new species isolated from human clinical specimens, the hospital environment and other sources. Intern. J. Syst. Bacteriol. 27, 133-146.
Watanabe I. et Barraquio W.L., 1979. Low levels of fixed nitrogen required for isolation of free-living N2-fixing organisms from rice roots. Nature (London), 277:565-566.
Weaver P.K., Wall J.D. et Gest H., 1975. Characterization of Rhodopseudomonas capsulata. Arch. Microbiol., 105,207-216.

**Revendications**

1) Procédé de préparation d'exopolysaccharides contenant moins de 10 % d'acide uronique, caractérisé en ce qu'on cultive sur un milieu de culture une souche identifiée comme Pseudomonas paucimobilis et en ce qu'on récupère les exopolysaccharides contenant moins de 10 % d'acide uronique du milieu de culture.

2) Procédé selon la revendication 1, caractérisé en ce que la source de carbone du milieu de culture est un aldohexose.

3) Procédé selon l'une des revendications 1 et 2, caractérisé en ce que la culture est effectuée en présence d'un prédateur.

4) Procédé selon la revendication 3, caractérisé en ce que le prédateur est une amibe du sol.

5) Procédé selon l'une des revendications 1 à 4, caractérisé en ce que la souche est la souche RMDP17.

6) Procédé selon l'une des revendications 1 à 5, caractérisé en ce que l'exopolysaccharide est récupéré par précipitation du milieu de culture.

7) Exopolysaccharide tel qu'il peut être obtenu par la mise en oeuvre du procédé selon l'une des revendications 1 à 6.

8) Exopolysaccharide pouvant être obtenu par le procédé selon la revendication 5.

9) Exopolysaccharide dont la chaîne saccharidique est la suivante :

$$\left[ 4\ \text{Glc}\ 1 \longrightarrow 3\ \underset{\substack{\uparrow 6 \\ \text{Glc Béta1}}}{\text{Glc Béta1}} \longrightarrow 6\ \text{Glc Alpha1} \longrightarrow 4\ \text{Rha}\ 1 \right]_n$$

10) Application des exopolysaccharides selon l'une des revendications 7 à 9 à titre d'agent épaississant et/ou agent de conditionnement des sols.

11) Souche de Pseudomonas paucimobilis fixatrice d'azote et qui donne, sur milieu dépourvu d'azote combiné, des colonies jaunes brillantes extrêment surélevées.

12) Application de la souche selon la revendication 11 comme agent de colonisation du mil et des plantes apparentées.

FIG_1

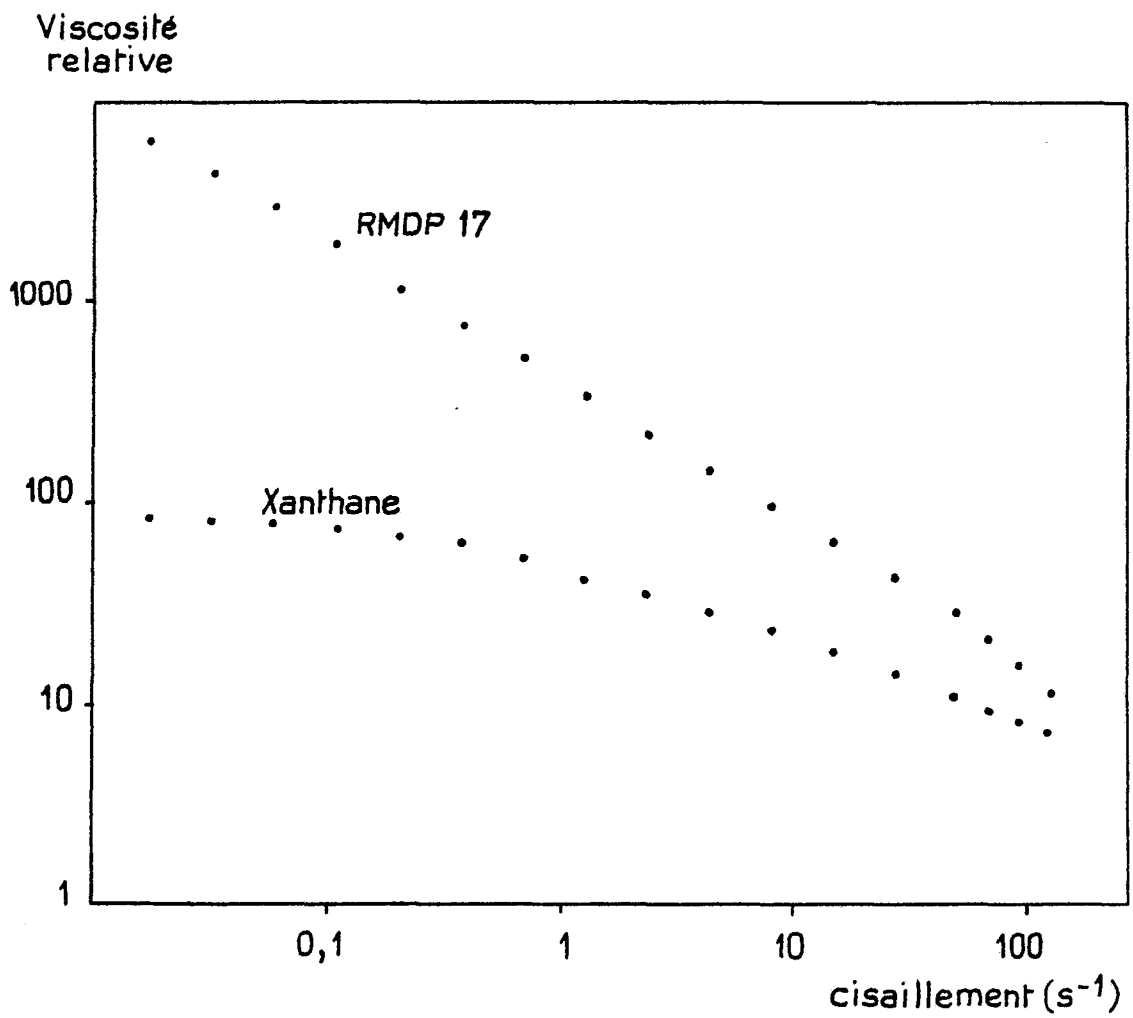
Viscosité relative
1000
100
10
1
RMDP 17
Xanthane
0,1
1
10
100
cisaillement (s-1)

FIG_2

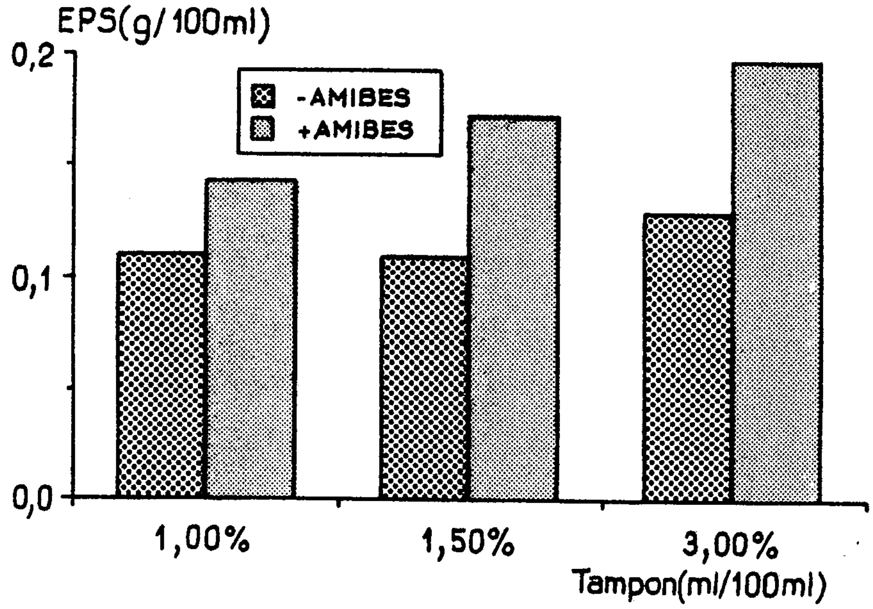
EPS(g/100ml)
0,2
0,1
0,0
-AMIBES
+AMIBES
1,00%
1,50%
3,00%
Tampon(ml/100ml)

FIG_3

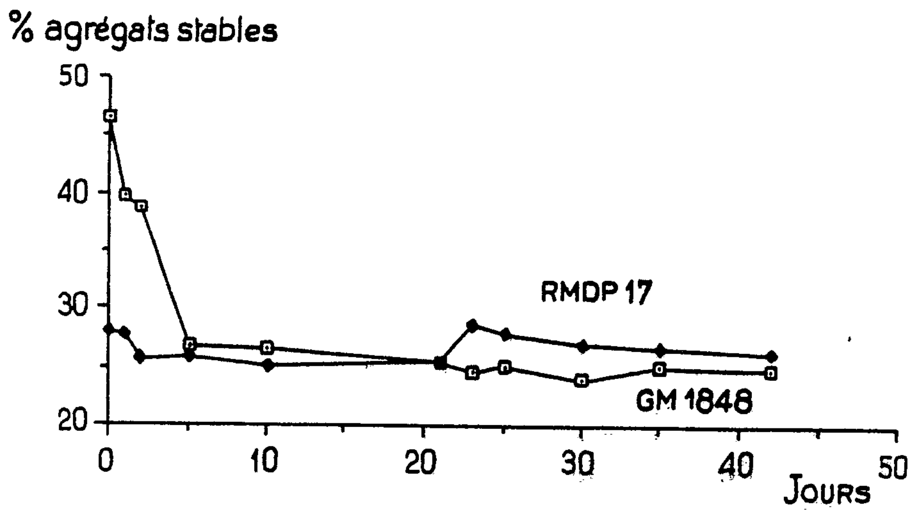
% agrégats stables
50
40
30
20
0
10
20
30
40
50
Jours
RMDP 17
GM 1848

Office européen des brevets

# RAPPORT DE RECHERCHE EUROPEENNE

Numero de la demande

EP 89 40 2105

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | JOURNAL OF APPLIED BACTERIOLOGY, vol. 62, 1987, pages 147-150, GB; A. ANSON et al.: "A bacterium yielding a polysaccharide with unusual properties" * En entier * | 1,2,7, 10 | C 12 P 19/04<br>C 12 N 1/20 //<br>C 12 R 1/38 |
| A | CHEMICAL ABSTRACTS, vol. 95, no. 21, 23 novembre 1981, page 333, abstracts no. 183694h, Columbus, Ohio, US; J.L. SINCLAIR et al.: "Nitrogen mineralization by Acanthamoeba polyphaga in grazed Pseudomonas paucimobilis populations", & APPL. ENVIRON, MICROBIOL. 1981, 42(4), 667-71 | 3-4 | |
| A | CHEMICAL ABSTRACTS, vol. 104, no. 11, 17 mars 1986, page 581, abstract no. 87594j, Columbus, Ohio, US; E.T. ELLIOTT et al.: "Short-term bacterial growth, nutrient uptake, and ATP turnover in sterilized, inoculated and carbon-amended soil: the influence of nitrogen availability", & SOIL BIOL. BIOCHEM. 1983, 15(1), 85-91 | | **DOMAINES TECHNIQUES RECHERCHES (Int. Cl.5)**<br>C 12 P |

Le présent rapport a été établi pour toutes les revendications

| Lieu de la recherche | Date d'achèvement de la recherche | Examinateur |
|---|---|---|
| LA HAYE | 06-11-1989 | LENSEN H.W.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant